# EUROPEAN PATENT APPLICATION

(11) **EP 3 662 934 A1**
(43) Date of publication of application: **10.06.2020**
(21) Application number: 18840364.6
(22) Date of filing: 12.07.2018
(51) Int. Cl.: A61K 48/00, C12N 15/11, C12N 15/66, A61P 25/00

(54) **COMPOSITION FOR GENE THERAPY OF THE CENTRAL NERVOUS SYSTEM, PROCESS OF PRODUCTION AND USE THEREOF**

(30) Priority: 31.07.2017 BR 102017016440
(71) Applicant: Universidade Federal Do Rio Grande Do Sul, 90040060 Porto Alegre-RS (BR)
(72) Inventor: SILVESTRI SCHUH, Roselena, 92110001 Porto Alegre (BR); FERREIRA TEIXEIRA, Helder, 90050-240 Porto Alegre (BR); BALDO, Guilherme, 90690-130 Porto Alegre (BR); DA SILVEIRA MATTE, Ursula, 90630000 Porto Alegre (BR); GIUGLIANI, Roberto, 90630080 Porto Alegre (BR); BIDONE, Juliana, 96020220 Porto Alegre (BR)
(74) Representative: Turri, Elisa
(86) International application number: PCT/BR2018/050236
(87) International publication number: WO 2019/023770

(57) **Abstract**

The present invention describes a composition for gene therapy of the central nervous system comprising non-viral carriers of nanometric size (<1.0 micrometer) complexed with at least one nucleic acid for purposes of gene therapy via nasal administration, having as main target the central nervous system and the processes for obtaining such carriers. The present invention belongs to the field of nanotechnology and consists of aqueous formulations that can be used in the pharmaceutical and medical fields.

## Description

### Field of the Invention

The present invention describes a composition for gene therapy of the central nervous system comprising non-viral carriers of nanometric size (< 1.0 micrometer) complexed with at least one nucleic acid for purposes of gene therapy via nasal administration having as main target the central nervous system, and in addition the processes for obtaining such carriers. The present invention belongs to the field of nanotechnology and consists of aqueous formulations that can be used in the pharmaceutical and medical fields.

### Background of the Invention

Deficiencies and/or genetic anomalies (mutation, aberrant expression, etc.) are involved in the origin of numerous diseases, hereditary or not. Conventional medicine is limited for treating these diseases, using therapies to relieve symptoms. More recently, gene therapy has emerged, which consists of inserting a functional gene in order to correct a cellular dysfunction or provide new functions to the cell, with the introduction of genetic material directly into the patient's cells (*in vivo*), or from the administration of cells after *in vitro* (*ex vivo*) modification. Gene therapy is defined as the genetic modification of cells with the intention of altering the expression of a gene to prevent, hinder or reverse a pathological process (KAY, MA State-of-the-art gene-based therapies: the road ahead. Nature Reviews Genetics 2011, v. 12, p. 316-328).

However, although promising, gene therapy faces several limitations related to the penetration capacity and intracellular stability of nucleic acids, due to its highly polyanionic character, the possibility of interaction and aggregation with proteins, and the occurrence of enzymatic degradation (LIU, C.-H.; YU, S.-Y. Cationic nanoemulsions as non-viral vectors for plasmid DNA delivery. Colloids and surfaces. B, Biointerfaces 2010, v. 79, n. 2, p. 509-515). In order to overcome these difficulties, some strategies have been used, such as the delivery of nucleic acids through association with viral and/or non-viral vectors.

The most commonly used viral vectors in gene therapy are adenoviruses, adeno-associated viruses, lentiviruses and retroviruses. Despite the great efficiency of insertion and transduction offered by viral vectors, they present some problems related to immunogenicity, replication and safety (YIN, H. et al. Non-viral vectors for gene-based therapy. Nature Reviews Genetics 2014, v. 15, n. 8, p. 541-545). In order to work around these problems, non-viral vectors are used, which have relative ease and low cost of large-scale production, less toxicity, low immunogenicity, ability to complex with high molecular weight nucleic acids, greater safety and good transfection capacity. (NAM, H. Y. et al. Lipid-based emulsion system as non-viral gene carriers. Archives of Pharmaceutical Research 2009, v. 32, n. 5, p. 639-646; NORDLING-DAVID, M. M.; GOLOMB, G. Gene Delivery by Liposomes Israel Journal of Chemistry 2013, v. 53, n. 9-10, SI, p. 737-747).

Transfection using non-viral vectors can occur through polymeric or lipid structures, the latter being more classic and safer regarding toxicity, biocompatibility and biodegradability of the biomaterials used. Among the vectors based on cationic lipids, the most described in the literature are liposomes, nanoemulsions, solid lipid nanoparticles and nanostructured lipid carriers. Cationic liposomes (NORDLING-DAVID, M. M.; GOLOMB, G. Gene Delivery by Liposomes. Israel Journal of Chemistry 2013, v. 53, n. 9-10, SI, p. 737-747) and cationic nanoemulsions (BRUXEL, F. et al. Investigation of the structural organization of cationic nanoemulsion/antisense oligonucleotide complexes. Colloids and surfaces B, Biointerfaces 2013, v. 112, p. 530-536) are among the most described non-viral lipid vectors. Liposomes can be defined as aqueous dispersions of a mixture of phospholipids, organized in the form of bilayers and with a central aqueous core. On the other hand, nanoemulsions, solid lipid nanoparticles and nanostructured lipid carriers are organized as monolayers with a respectively liquid, solid lipid core, or both, dispersed in an aqueous phase (usually of the O/W type), and stabilized by an interfacial film constituted by phospholipid emulsifiers (SCHUH, R. S.; BRUXEL, F.; TEIXEIRA, H. F. Physicochemical properties of lecithin-based nanoemulsions obtained by spontaneous emulsification or high-pressure homogenization. Química Nova 2014, v. 37, p. 1193-1198).

Regardless of the structure formed, these non-viral systems contain a cationic lipid (usually a quaternary amine) that forms an ionic pair (complex) with the negatively charged phosphate groups of nucleic acids. Several studies demonstrate the efficiency of these complexes formed by lipid nanostructures/nucleic acids (NORDLING-DAVID, M. M.; GOLOMB, G. Gene Delivery by Liposomes. Israel Journal of Chemistry 2013, v. 53, n. 9-10, SI, p. 737-747; FRAGA, M. et al. PEGylated cationic nanoemulsions can efficiently bind and transfect pIDUA in a mucopolysaccharidosis type I murine model. Journal of Controlled Release 2015, v. 209, p. 37-46). However, some limitations related to the release of nucleic acids *in vivo,* due to the capture of complexes by the mononuclear phagocytic system and its limited biodistribution, require some formulation strategies. Among these, the incorporation of phospholipids covalently linked to hydrophilic polymers such as polyethylene glycol (PEG) seems to confer a longer circulation time to the complexes and a greater protection from nucleic acids, which makes it possible to increase their biodistribution in tissues and, consequently, efficiency of transfection (FRAGA, M. et al. PEGylated cationic nanoemulsions can efficiently bind and transfect pIDUA in a mucopolysaccharidosis type I murine model. Journal of Controlled Release 2015, v. 209, p. 37-46).

In addition, the use of polycations such as chitosan in formulations for administration is widespread, especially due to their mucus adhesive properties, especially when the target is nasal administration aiming for the treatment of disorders of the central nervous system (Khatri, K. et al. Surface modified liposomes for nasal delivery of DNA vaccine. Vaccine, 2008, V. 26(18), p. 2225-33).

The possibilities for treating diseases generated by gene therapy are numerous, and their carrying through non-viral vectors greatly increases the chances of success, however the arrival of these compositions in the central nervous system remains a challenge. The brain is an exclusively protected organ that resides within the osseous limits of the skull, making it difficult to reach through systemic drug delivery. A variety of obstacles protects the central nervous system while preventing medications from reaching the brain and spinal cord and include the blood-brain barrier (BBB) and the blood-cerebrospinal fluid barrier (BCSFB). Blood-brain barriers restrict the passive diffusion of macromolecules to the brain and constitute a significant obstacle to the brain/central nervous system (CNS) in the pharmacological treatment of genetic diseases with neurological impairment, including lysosomal deposit diseases (Saraiva, C. et al. Nanoparticle-mediated brain drug delivery: Overcoming blood-brain barrier to treat neurodegenerative diseases. Journal of Controlled Release, 2016, v. 235, p. 34-47).

Invasive methods of CNS treatment include direct intracranial drug administration by intracerebroventricular, intracerebral or intrathecal administration, and create holes in the head that disrupt the integrity of the blood-brain barrier by osmotic rupture of the blood brain barrier.

Thus, the nasal route started to be explored as a non-invasive method to work around the BBB for the transport of drugs to the CNS and has been proven effective for several small molecules and peptides. This route of drug administration works due to the unique neuronal connection that the trigeminal and olfactory nerves have between the nasal cavity, cerebrospinal fluid (CSF) and the brain.

In the search for the state of the art in scientific and patent literature, the following documents dealing with the topic were found:

The technologies protected by the numbers WO 2015089419 (A2) 18/06/2015, and WO2014093622 (A2) 06/19/2014, describe the use of particles for delivery of the CRISPR/Cas system. The liposomes from the protected technologies are produced by a method of extrusion through membrane or spontaneous formation by hydrating the lipid film (Coelho et al, N Engl J Med 2013, v. 369, p. 819-29; Basha et al, Molecular Therapy 2011, v. 19(12), p. 1286-00; Morrissey et al, Nature Biotechnology 2005, v. 23(8), p. 1002-07; Zimrnerrnan et al, Nature Letters 2006, v. 441(4), p. 111-14; Geisbert et al, Lancet 2010, v. 375, p. 1896-905; Semple et al, Nature Nanotechnology 2010, v. 28(2), p. 172-177; Jayararnan A. , Chem. Int. Ed. 2012, v. 51, p. 8529-33; U.S. Pat. Nos. 5593972, 5589466 and 5580859). The protected technology also cites nanoplexes (Bartlett et al, PNAS 2007, v. 104(39), p. 15549-54) and a nanoparticle-based delivery system (Davis et al, Nature 2010, v. 464(15), pp. 1067-70), which use cyclodextrins in their composition, differing from the present invention. The cited nanoparticles contain polymers, differing from the present invention.

The WO 2016197133 (A1) technology describes how to deliver the CRISPR system with lipid nanoparticles but does not describe complexing with two different nucleic acid sequences or proteins.

The technology protected under the number WO 2015191693 (A2) 12/17/2015, proposes the use of two different vectors, one carrying the guide RNA and the other the genome editing system, in addition to liposomes and polymeric nanoparticles produced by different methods from those mentioned in the present invention.

The technology protected under WO 2015US23882 (A2) describes methods and compositions for the prevention or treatment of disorders of the central nervous system, however it does not describe the use of lipid carriers for this purpose.

The technology EP 3087974 (A1) describes nanocarriers for delivery of a genome-editing composition, however it only mentions liposomes and micelles, and these have a binding molecule of a specific receptor.

The technology protected under WO 2015089462 (A1) describes lipid nanoparticulate compositions for CRISPR delivery, however it is composed only of RNA molecules and does not mention compositions containing different nucleic acids and proteins. It also determines a lipid:gRNA ratio from 5:1 to 15:1, different from the propositions of the present invention.

The technology WO 2013188979 (A1) refers, in general, to mucoadhesive nanoparticles formed from polymeric amphiphilic macromolecules conjugated to a polymeric coating for drug delivery in general but does not use lipids in its main composition.

The IN2011MU01507 technology presents a pharmaceutical composition comprising a drug or drug vehicle that after intranasal administration leads to an improvement in receptor mediated brain uptake of the drug but does not deal with the delivery of nucleic acids.

The technology protected under WO 200641942 (A2) describes a composition that can be used as a biodegradable implant.

The WO2016174250 (A1) technology refers to nanocarriers with anchoring ligands to deliver a tool for gene transfer to cells. The anchors have a targeting portion that can be a carbohydrate, an antibody or an antibody fragment, a protein, an aptamer, among others.

The technology protected under number WO2015179492 (A1) demonstrates processes for the preparation of polymeric nanoparticles containing nucleic acids for the treatment of neurological diseases. This process does not use lipid components in its production.

The technology WO2015117021 (A1) refers in part to methods for delivery of nucleic acids but has the skin as main target.

The WO2012135805 (A1) technology describes a pharmaceutical composition for delivery of polynucleotides but does not determine the delivery of two nucleic acid sequences concurrently.

Thus, from what can be inferred from the researched literature, no documents were found anticipating or suggesting the teachings of the present invention, so that the solution proposed here has novelty and inventive activity in view of the state of the art.

### Summary of the Invention

Thus, the present invention differs from the state of the art, comprising the use of four different types of aqueous nanometric carriers, produced by methods other than those mentioned in the state of the art, containing at least one complexed nucleic acid in the same formulation, for nasal administration having the CNS as target for gene therapy purposes.

The technology described in the present invention provides new compositions and methods for treating syndromes that primarily affect the central nervous system. In some realizations of the following invention, it can be administered from once a day to several times a day, for several days.

In some realizations, compositions for gene therapy of the central nervous system can be administered as intranasal or intratracheal spray for cerebral delivery, by inhalation, and/or through other aerosol vehicles.

The present invention also presents the incorporation of a plasmid of the CRISPR/Cas9 system together with another nucleic acid.

The present invention also features the incorporation of an encoding recombinant plasmid for a protein.

In a first object, the present invention presents a composition for gene therapy of the central nervous system comprising at least one adsorbed or encapsulated nucleic acid and non-viral carriers, with average droplet/particle diameter ranging from 0.001 to 1.0 micrometer.

In a second object, the present invention presents a process for obtaining composition for gene therapy of the central nervous system, where obtaining non-viral carriers comprises the steps of:
(a) dissolving between 2.0% w/w to 20.0% w/w of lipid phase in an organic solution;
(b) dissolving between 0.1% w/w to 5.0% w/w of tonicity agent in an aqueous solution;
(c)evaporating the organic solution obtained in step (a), to form a film;
(d) adding the aqueous solution obtained in step (b) to the lipid film obtained in step (c);
(e) letting the product obtained in step (d) rest for 4 to 72 hours at a temperature between 2°C and 20°C;
(f) sonicating the formulation obtained in step (e) for 1 to 60 minutes at a temperature between 25°C and 50°C;
(g) homogenizing the formulation obtained in step (f) in a high-pressure homogenizer or microfluidizer, for 2 to 20 cycles of 250 to 2000 bar each;
(h) nucleic acids for proportions between +2/-1 and +10/-1 (DOTAP/NUCLEIC ACID); and
(i) adding a polycation solution with a concentration of 0.001 mg/mL to 10 mg/mL.

In a third object, the present invention presents a process for obtaining a composition for gene therapy of the central nervous system to obtain non-viral carriers, including solid lipid nanostructures and nanostructured lipid carriers containing the adsorbed nucleic acids, comprising the steps of:
(a) melting from 2.0% w/w to 20.0% w/w of lipid phase at a temperature between 30°C and 80°C;
(b) dissolving from 1.0% w/w to 5% w/w surfactant and 0.1% w/w 5.0% w/w of a tonicity agent in an aqueous solution, with a temperature of 30°C to 80°C;
(c)adding the aqueous solution from step (A) to the oily solution from step (B), under stirring and at a temperature of 30°C to 80°C;
(d) stirring the product obtained in (C) in disperser ultra-turrax, at a speed between 500 and 25000 rpm, under heating from 30°C to 80°C, for 30 seconds to 10 minutes; and
(e) homogenizing the formulation obtained in (D) in a homogenizer at high pressure or microfluidizer, for 2 to 20 cycles of 250 to 2000 bar each;
(f) nucleic acids for proportions between +2/-1 and +10/-1 (DOTAP/NUCLEIC ACID); and
(g) adding a polycation solution with a concentration of 0.001 mg/mL to 10 mg/mL.

In a fourth object, the present invention presents the use of the composition for gene therapy of the central nervous system in the preparation of a medication for the treatment of diseases caused by deficiencies or genetic abnormalities such as lysosomal deposit diseases.

In addition, the inventive concepts common to all protection contexts claim a composition for gene therapy of the central nervous system, comprising non-viral carriers of nanometric size and at least one nucleic acid adsorbed or encapsulated with average droplet/particle diameter in the range from 0.001 to 1.0 micrometer.

In addition, the compositions may be incorporated in the form of a solution, suspension, gel, powder, among others.

These and other objects of the invention will be immediately valued by those skilled in the art and by companies with interests in the segment and will be described in enough detail for their reproduction in the following description.

### Brief Description of the Drawings

In order to better define and clarify the content of this patent application, the following figures are presented:

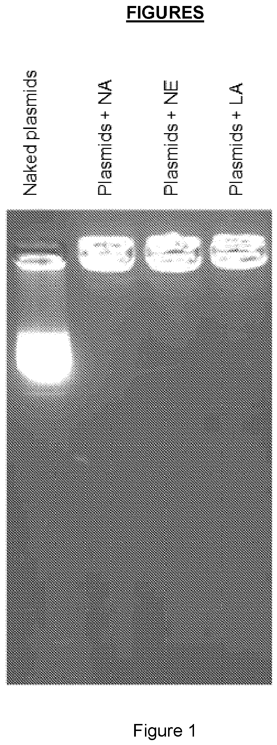

Figure 1 demonstrates the co-complexation of the formulations with a plasmid from the CRISPR/Cas9 system and a plasmid donor of the sequence of the enzyme alpha-L-iduronidase (IDUA) used to repair the genome by homologous recombination after cleavage by Cas9, directed to the locus Pink 26 of mice. Bands of the naked plasmids can be observed, and it's possible to see the nanoemulsion formulations with adsorbed nucleic acids (NA), nanoemulsion with encapsulated nucleic acids (NE) and liposomes with adsorbed nucleic acids (LA) complexed the plasmids that did not migrate in the gel, showing 100% complexation as they remained at the point of application. The 100% rate was calculated using the ImageJ® software.
Figure 2 shows the enzyme activity values of murine IDUA found in the serum of untreated MPS I mice, and in MPS I mice treated with the LA CRISPR/pROSA26 complex or with the CRISPR/pROSA26 naked plasmids, nasally for 15 days. Values related to the enzymatic activity of normal mice.
Figure 3 shows the enzyme activity values of murine IDUA found in different brain sections of untreated MPS I mice and in MPS I mice treated with the LA CRISPR/pROSA26 complex or with the CRISPR/pROSA26 naked plasmids nasally for 30 days. Values related to the enzymatic activity of normal mice.

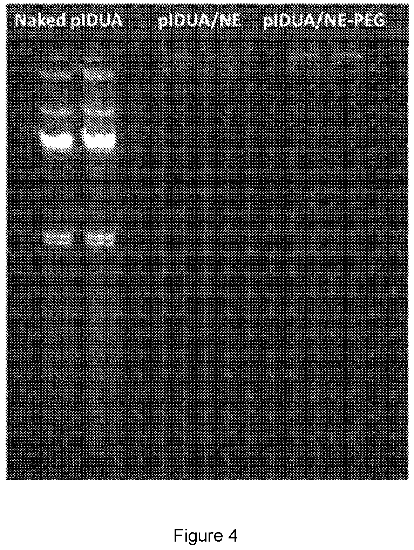

Figure 4 demonstrates the co-complexation of the formulations with a plasmid (pIDUA) containing the cDNA of IDUA constructed using the commercial expression vector pREP9 (Invitrogen, USA) as described by Camassola and collaborators (M. Camassola, L.M. Braga, A. Delgado-Cañedo, T.P. Dalberto, U. Matte, M. Burin, R. Giugliani, N.B. Nardi, Nonviral in vivo gene transfer in the mucopolysaccharidosis I murine model, J. Inherit. Metab. Dis. 28 (2005) 1035-1043). Bands of naked plasmids can be observed, and it's possible to see that the nanoemulsion formulations with adsorbed nucleic acids (NA) and the nanoemulsion with encapsulated nucleic acids (NE) complexed the plasmids that did not migrate in the gel, demonstrating 100% complexation because they remained at the point of application. The 100% rate was calculated using the ImageJ® software.
Figure 5 shows the IDUA enzyme activity values found in different organs and more precisely in the brain of untreated MPS I mice and in MPS I mice treated with the NA/pIDUA complex nasally in one application. Values related to the enzymatic activity of normal mice.

### Detailed Description of the Invention

Gene therapy allows an organism to produce a deficient protein that is essential for its proper functioning through the administration of nucleic acid sequences that code for the protein in question. For this purpose, it is possible to use a recombinant plasmid that has the correct sequence of the abnormal protein and can overexpress it or it is possible to use gene editing technologies. In preferred embodiments, the recombinant plasmid is complexed to a carrier that will be administered via the nasal route.

The genome editing technology makes it possible to modify specific sequences of the genome through the recognition of the region to be changed and the use of nucleases capable of cleaving at the target site. Genomic manipulation has raised expectations, as it makes it possible to aim for any target gene, and thus increases the chances of treatment for genetic diseases. For this, systems composed of a domain of recognition and binding to specific sequences of genomic DNA are used together with a domain of cleavage of the target sequence in the DNA (COX, D.B.T.; PLATT, RJ.; ZHANG, F. Therapeutic genome editing: prospects and challenges. Nature Medicine 2015, v. 21, n. 2, p. 121-131).

Genome editing platforms are based on nuclease proteins targeted to cleave target sites in the genome. The nuclease can be a transcription-activating effector nuclease (TALEN), a zinc finger nuclease (ZFN), a meganuclease or a CRISPR-associated nuclease (Cas). In some embodiments, these nucleases are delivered in the form of nucleic acid sequences (plasmids or oligonucleotides) encoding for these proteins. In some embodiments, the protein is a CRISPR-associated nuclease and is provided as part of a ribonucleoprotein (RNP) that includes a recombinant Cas9 protein combined with guide RNA (gRNA), which guides the nuclease to the target site of cleavage in the genome.

The nucleic acid to be delivered may be the DNA of a plasmideal vector, the messenger RNA (mRNA) or the gRNA that encodes an enzyme or is part of the enzyme that will act by cleaving the target genetic material, or it may be a model sequence used for repairing the target genome by homologous recombination. Where the target in the genome includes any sequence that can be modified to promote protein silencing, expression or overexpression. In preferred embodiments, the nucleic acid will be complexed with a lipid carrier that will be administered via the nasal route.

The targetable nuclease can be a transcription-activating effector nuclease (TALEN), a zinc finger nuclease (ZFN), a meganuclease or a CRISPR-associated nuclease (Cas). In some embodiments, the targetable nuclease is a CRISPR-associated nuclease and is supplied as part of an RNP that includes a recombinant Cas9 protein combined with the gRNA. In preferred embodiments, the targetable nuclease is complexed with a carrier that will be administered via the nasal route.

However, some diseases have CNS involvement and require treatment to reach the brain. For the administration of non-viral vectors containing a recombinant plasmid or the CRISPR/Cas9 system aimed at gene therapy of diseases with CNS involvement, the nasal route is suggested, which is a highly vascularized, easily accessible and non-invasive region. In addition to be a route evaluated for systemic absorption, more recently it has been studied as a route of direct passage of molecules to the brain (GHORI, M. U. et al. Nasal Drug Delivery Systems: An Overview. American Journal of Pharmacological Sciences, v. 3, n. 5, p. 110-119, Dec. 18, 2015.). There are several reports of the satisfactory passage of macromolecules through the blood-brain barrier after nasal administration. This route involves the olfactory system that begins in the brain and ends in the nasal cavity, in the respiratory epithelium, being the only region of the central nervous system considered to be easily accessible (LOCHHEAD, J. J.; THORNE, R. G. Intranasal delivery of biologics to the central nervous system. Advanced drug delivery reviews, v. 64, n. 7, p. 614-628, May 2012).

For the carriers to reach mainly the nervous system, nasal administration can occur through intranasal or intratracheal spray, by inhalation, and/or through other aerosol vehicles. In addition, the compositions may be incorporated in the form of a solution, suspension, gel, powder, among others.

In this way, the invention provides methods and compositions that allow the production of a deficient protein by an individual through the nasal administration of non-viral carriers containing a nucleic acid sequence that encodes a protein or even a nuclease that cleaves the target genetic material.

In view of the above, considering the low intracellular penetrability of naked nucleic acids, together with the advantages of using nanometric systems in the carrying and administration of nucleic acids, together with the biological potential of the administration of these complexes, the present invention refers to aqueous formulations comprising at least one nucleic acid complexed to non-viral carriers with an average droplet/particle diameter less than 1.0 micrometer. The nanocarriers of the present invention comprise nanoemulsions, liposomes, solid lipid nanoparticles and nanostructured lipid carriers.

The manufacturing process of the products comprises a high-pressure homogenization or microfluidization step, in order to produce uniformly sized and highly stable nanometric lipid carriers. In addition, the manufacturing process for nanoemulsions may comprise a pre-complexation step with nucleic acids, which provides greater protection against degradation. The liposome manufacturing process, on the other hand, goes through an additional step of manual extrusion that gives high stability to the products. Carriers containing at least one nucleic acid for genome editing should be used preferably by nasal administration.

In a first object, the present invention presents a composition for gene therapy of the central nervous system comprising at least one adsorbed or encapsulated nucleic acid and non-viral carriers, with average droplet/particle diameter ranging from 0.001 to 1.0 micrometer.

In one embodiment, the nucleic acids are one or more selected from the group consisting of: recombinant plasmid containing the entire sequence of a gene, guide RNA sequence, nuclease coding sequence, model DNA sequence for homologous recombination or entire sequence of a gene.

In one embodiment, the central nervous system gene therapy composition comprises a nuclease, which may be Cas9.

In one embodiment, nanostructures are nanoemulsions with adsorbed or encapsulated nucleic acids, liposomes, solid lipid nanoparticles or nanostructured lipid carriers.

In one embodiment, the composition for central nervous system gene therapy comprises pharmaceutically suitable excipients.

In a second object, the present invention presents a process of obtaining composition for gene therapy of the central nervous system, where obtaining carriers comprises the steps of:
(a) dissolving between 2.0% w/w to 20.0% w/w of lipid phase in an organic solution;
(b) dissolving between 0.1% w/w to 5.0% w/w of tonicity agent in an aqueous solution;
(c)evaporating the organic solution obtained in step (a), to form a film;
(d) adding the aqueous solution obtained in step (b) to the lipid film obtained in step (c);
(e) letting the product obtained in step (d) rest for 4 to 72 hours at a temperature between 2°C and 20°C;
(f) sonicating the formulation obtained in step (e) for 1 to 60 minutes at a temperature between 25°C and 50°C;
(g) homogenizing the formulation obtained in step (f) in a high-pressure homogenizer or microfluidizer, for 2 to 20 cycles of 250 to 2000 bar each; and
(h) adding a solution of polycations with a concentration of 0.001 mg/mL to 10 mg/mL.

In one embodiment, the organic solution described in step (a) is an non-polar organic solvent.

In one embodiment, if the product to be obtained is the liposome, the process comprises the additional step:
(i) extruding the formulation obtained in step (g) on at least one membrane with pore size from 1000 nm to 220 nm and on at least one membrane with pore size from 220 nm to 50 nm.

In one embodiment, the organic solution is an organic solvent chosen from the group comprising protic, aprotic or non-polar polar organic solvents and/or a mixture thereof.

In one embodiment, a solution of non-lipid polycations can be added after the formation of the nanostructures.

In one embodiment, to obtain nanoemulsions containing encapsulated nucleic acids, the organic solution described in step (a) is the organic phase of the pre-complex obtained through the steps:
(i) dissolving from 0.1% w/w to 5.0% w/w of cationic lipid and nucleic acids in a monophasic mixture of nonpolar:protic:protic solvents (1:2.1:1) for 30 minutes;
(ii) adding to the product obtained in step (i) 2 mL of non-polar solvent and 2 mL of protic solvent;
(iii) stirring the product obtained in step (ii) briefly in vortex;
(iv) centrifuging the product obtained in step (iii) at a pressure between 1000 and 4000 x g for 2 to 30 min at a temperature between 15 to 35°C; and
(v) separating the organic phase obtained in step (iv)

In a third object, the present invention presents a process for obtaining a composition for gene therapy of the central nervous system to obtain solid lipid nanostructures or nanostructured lipid carriers containing the adsorbed nucleic acids, comprising the steps of:
(A) melting from 2.0% w/w to 20.0% w/w of lipid phase at a temperature between 30°C and 80°C;
(B) dissolving 1.0% w/w 5% w/w surfactant and from 0.1% w/w to 5.0% w/w of a tonicity agent in an aqueous solution, with a temperature of 30°C to 80°C;
(C) adding the aqueous solution from step (A) to the oily solution from step (B), under stirring and at a temperature of 30°C to 80°C;
(D) stirring the product obtained in (C) in disperser ultra-turrax, at a speed between 500 and 25000 rpm, under heating from 30°C to 80°C, for 30 seconds to 5 minutes;
(E) homogenizing the formulation obtained in (D) in a homogenizer at high pressure or microfluidizer, for 2 to 20 cycles of 250 to 2000 bar each; and
(F) adding a polycation solution with a concentration of 0.001 mg/mL to 10 mg/mL.

In one embodiment, the formulation is subjected to a later stage of water evaporation under normal or reduced pressure between 0 and 1000 mbar at a temperature between 10°C and 50°C.

In one embodiment, the protic polar organic solvent is methanol, and the non-polar organic solvent is chloroform.

In one embodiment, a solution of non-lipid polycations can be added after the formation of lipid nanostructures.

In one embodiment, the lipid phase is chosen from the group comprising:
a) liquid lipids such as decyl oleate, isohexadecane, stearic and/or oleic acid esters, coconut fatty acid ethanolamide, natural oils such as corn oil, peanuts, sesame, olive, jojoba, soy, fatty alcohol, paraffin, medium chain triglycerides, long chain triglycerides, palmitates, myristates and octyldodecanol;
b) solid lipids such as tristearin, tricaprine, trilaurine, trimiristin, tripalmitin, stearic acid, cetyl alcohol, stearyl alcohol, cocoa mate, carnauba wax, beeswax, cetyl palmitate, glycerol monostearate, glycerol behenate, glyceryl palmitostearate, glyceryl tripalmitate, glyceryl trimiristate, glyceryl tristearate and/or a mixture thereof;
c) lipophilic surfactants such as lecithins and phospholipids and/or a mixture thereof;
d) neutral lipids;
e) cationic lipids; and
f) lipids with PEG branching (pegylated).

In one embodiment, the tonicity agent will be chosen from the group comprising sorbitol, ethylene glycol, polyethylene glycol, mannitol, glycerol, and/or a mixture thereof.

In one embodiment, the solution of non-lipid polycations at a concentration of 0.001 mg/mL (w/v) to 10 mg/mL (w/v), comprises chitosan, hexadimethrin bromide or another salt, poly-L-lysine, polyalylamine, polyethyleneimine, among others, and/or a mixture of these. The addition of these can be carried out after the formation of lipid nanostructures or complexes with nucleic acids.

In one embodiment, the lipid phase and the aqueous phase of the liposome obtaining process comprise:
Lipid phase:
   - DOPE (0.5% w/w to 5.0% w/w);
   - DOTAP (0.5% w/w to 5.0% w/w);
   - DSPE-PEG (0.25% w/w to 5.0% w/w);
Aqueous phase:
   - Glycerol (0.1% w/w to 5.0% w/w);
   - Nucleic acids for the ratio between +2/-1 and +8/-1 (DOTAP/NUCLEIC ACID);
   - Chitosan solution (0.001 mg/mL to 10 mg/mL).

In one embodiment, the lipid phase and the aqueous phase of the process for obtaining the nanoemulsions comprise:
Lipid phase:
   - DOPE (0.5% w/w to 5.0% w/w);
   - DOTAP (0.5% w/w to 5.0% w/w);
   - DSPE-PEG (0.25% w/w to 5.0% w/w);
   - Medium chain triglycerides (2.0% w/w to 20.0% w/w);
Aqueous phase:
   - Glycerol (0.1% w/w and 5.0% w/w);
   - Nucleic acids for the ratio between +2/-1 and +8/-1 (DOTAP/NUCLEIC ACID);
   - Chitosan solution (0.001 mg/mL to 10 mg/mL).

In one embodiment, the lipid phase and the aqueous phase of the process for obtaining solid lipid nanoparticles comprise:
Lipid phase:
   - glyceryl monostearate (2.0% w/w to 10.0% w/w);
   - DOTAP (0.5% w/w to 5.0% w/w);
Aqueous phase:
   - 1.0% w/w Polysorbate 80 (1.0% w/w to 5.0% w/w);
   - Glycerol (0.1% w/w and 5.0% w/w);
   - Nucleic acids for the ratio between +2/-1 and +8/-1 (DOTAP/NUCLEIC ACID);
   - Chitosan solution (0.001 mg/mL to 10 mg/mL).

In one embodiment, the lipid and aqueous phases of the process for obtaining nanostructured lipid carriers include:
Lipid phase:
   - Mixing in the proportion 7:3 of glyceryl monostearate and medium chain triglycerides (2.0% w/w to 10.0% w/w);
   - DOTAP (0.5% w/w to 5.0% w/w);
Aqueous phase:
   - Polysorbate 80 (1.0% w/w to 5.0% w/w);
   - Glycerol (0.1% w/w and 5.0% w/w);
   - Nucleic acids for the proportion between +2/-1 and +8/-1 (DOTAP/NUCLEIC ACID);
   - Chitosan solution (0.001 mg/mL to 10 mg/mL).

In one embodiment, a solution of non-lipid polycations can be added after the formation of the nanostructures.

In one embodiment, the compositions may be incorporated in the form of a solution, suspension, gel, powder, among others.

In a fourth object, the present invention presents the use of the composition for gene therapy of the central nervous system in the preparation of a drug for the treatment of diseases caused by deficiencies or genetic abnormalities.

In one embodiment, the use of the composition for gene therapy of the central nervous system is in the preparation of a drug for the treatment of lysosomal deposit diseases that have neurological involvement.

In an ideal embodiment, the use of the composition for gene therapy of the central nervous system is through nasal administration.

The present invention has as advantages a greater intracellular penetrability due to the use of nanometric systems in the transport and administration of nucleic acids enabling the production of a deficient protein, through the use of nucleases combined with guide nucleic acids and nucleic acids containing the partial or entire sequence of a gene, or a recombinant plasmid containing the entire sequence of a gene. Also, an advantage is the possibility of treating diseases that may be caused by genomic problems using the products of the present invention.

### Examples - Embodiments

The examples shown herein are intended only to exemplify one of the countless ways of carrying out the invention, however without limiting its scope.

### Nucleic acids

The composition for central nervous system gene therapy must contain at least one nucleic acid, be it a guide RNA sequence, a plasmid or oligonucleotide containing the coding sequence for the Cas protein or another nuclease, a donor DNA sequence for homologous recombination or yet an entire sequence of a gene that may or may not be contained in a recombinant plasmid. Protein nuclease can also be part of the central nervous system gene therapy composition.

In the compositions of the present invention, the nucleic acid can be either a deoxyribonucleic acid or a ribonucleic acid. It may be a sequence of natural or artificial origin.

More particularly, regarding to the deoxyribonucleic acids, they can be single or double-stranded. These deoxyribonucleic acids can code for enzymes, mRNA or partial sequences or entire therapeutic genes.

In the sense of the invention, a therapeutic gene is understood to mean any gene encoding for a proteic product having a therapeutic effect. The proteic product thus encoded can be a protein, a peptide, etc. This protein product can be homologous with respect to the target cell (that is, a product that is normally expressed in the target cell, when it has no pathology). In that case, the expression of a protein allows, for example, to palliate an insufficient expression in the cell, or the expression of an inactive or weakly active protein due to a modification, or to overexpress said protein. The therapeutic gene can also code for a mutant cell protein, having increased stability, modified activity, etc. The proteic product can also be heterologous with respect to the target cell. In this case, an expressed protein can, for example, complete or promote a defective activity for the cell, allowing it to fight a pathology, or to stimulate an immune response.

### Lipid phase

The lipid phase suitable for the present invention consists of lipophilic surfactants, oils, solid and liquid lipids and/or a mixture thereof.

Lipid surfactants include, but are not limited to, lecithin and phospholipids. Lecithins are known as glycerophospholipids which are formed from fatty acids, glycerol, phosphoric acid and choline by esterification. Lecithins are often referred to as phosphatidylcholines. Phospholipids suitable for use in the present invention include, but are not limited to, phospholipids found in egg yolk and soy. Examples of phospholipids and their derivatives, phosphatidylcholine (PC), dioleylphosphatidylcholine (DOPC), dimyristoylphosphatidylcholine (DMPC), dipalmitoylphosphatidylcholine (DPPC), diestearoylphosphatidylcholine (DSPC), phosphatidylethanolamine (PE) dioleylphosphatidylethanolamine (DOPE), distearoylphosphatidylethanolamine (DSPE), phosphatidylserine (PS), dimiristoylphosphatidylglycerol (DMPG), dipalmitoylphosphatidylglycerol (DPPG), phosphatidyl inositol (PI), dipalmitoylphosphatidylserine (DPPS), distearoylphosphatidylserine (DSPS).

Oily substances suitable for use in the present invention include, but are not limited to, decyl oleate, isohexadecane, stearic and/or oleic acid esters, coconut fatty acid ethanolamide, natural oils such as corn oil, peanuts, sesame, olive, jojoba, soy, fatty alcohol, paraffin, medium chain triglycerides, long chain triglycerides, palmitates, myristates and octyldodecanol.

Solid lipids suitable for use in the present invention include, but are not limited to, triglycerides (tristearin, tricaprine, trilaurine, trimiristin, tripalmitin), fatty acids (stearic acid), fatty alcohols (cetyl alcohol, stearyl alcohol), waxes (cocoa butter, carnauba wax, beeswax, cetyl palmitate), partial glycids (glyceryl monostearate, glyceryl behenate, glyceryl palmitostearate, glyceryl tripalmitate, glyceryl trimiristate, glyceryl tristearate) and/or mixture thereof.

Lipids can be pegylated, that is, having a branch of polyethylene glycol (PEG) in its chain, such as DSPE-PEG, DMPE-PEG, cholesterol-PEG, DPPE-PEG (dipalmitoylglycerophosphoethanolamina-polyethylene glycol), DLPE-PEG (dilauroylglycerophosphoethanolamine-polyethylene glycol), among others.

Lipids can be cationic, such as 1,2-di-ortho-octadecenyl-3-trimethylammoniumpropane (DOTMA), 1,2-dimiristoleil-sn-glycero-3-ethylphosphocholine (EPC), didodecyldimethylammonium (DDAB), 1, dimethyldioctadecylammonium (DODAP), dioleyltrimethylammonopropane (DOTAP), dimethylaminoethanocarbamoyl cholesterol (DC-cholesterol), among others.

In order to obtain an optimal effect of the compositions of the invention, the respective proportions of nucleic acid and cationic lipid are preferably determined in such a way that the ratio of positive charges of the transfection agent/negative charges of the nucleic acids is comprised between 0,1 and 15 and, more preferably, between 2 and 8. This charge ratio may or may not account for other positively or negatively charged lipids in the formulation.

Most preferably, the compositions of the invention comprise, in addition, one or several neutral lipids. The applicant predicts that the addition of a neutral lipid allows the improvement of the formation of lipid particles and, surprisingly, favors the penetration of the particle into the cell, destabilizing its membrane. In a particularly advantageous way, natural or synthetic lipids, zwitterionic or devoid of ionic charge are used under physiological conditions. They can be chosen more particularly from dioleylphosphatidylethanolamine (DOPE), oleylpalmitoylphosphatidylethanolamine (POPE), di-stearoyl, - palmitoyl, - miristoyl phosphatidyl-5 nolamine, as well as their N-methylated derivatives 1 to 3 times; phosphatidylglycerols, diacylglycerols, glycosyl diacylglycerols, cerebrosides (such as galactocerebrosidium, notably), sphingolipids (such as sphingomyelins, notably), or asialogangliosides (such as asialoGM1 and Glv12, notably).

Preferably, the compositions of the invention, which employ a lipofectant as a transfection agent, comprise a ratio of 0,1 to 20 equivalents of neutral lipid to 0,1 to 20 equivalents of cationic lipid, and, more preferably, the ratio is respectively 1 to 5 to 1 to 5, respectively.

In the case where the transfection agent is a cationic lipid, the compositions of the invention comprise, in addition to the cationic lipid in the ratios mentioned above, from 0,1 to 20 molar equivalents of neutral lipid to 1 molar equivalent of nucleic acid phosphate, and, most preferably from 2 to 8.

In a preferred embodiment the liposomes described in the present invention comprise the use of DOPE (0.5% w/w 5.0% w/w), DOTAP (0.5% w/w 5.0% w/w) and DSPE-PEG (0.25% w/w to 5.0% w/w).

In a preferred embodiment, the nanoemulsions described in the present invention comprise the use of DOPE (0.5% w/w 5.0% w/w), DOTAP (0.5% w/w 5.0% w/w), DSPE-PEG (0.25% w/w 5.0% w/w) and medium chain triglycerides (2.0% w/w 20.0% w/w).

In a preferred embodiment, the solid lipid nanoparticles described in the present invention comprise the use of glyceryl monostearate (2.0% w/w to 10.0% w/w).

In a preferred embodiment, the nanostructured lipid carriers described in the present invention comprise the use of a 7:3 mixture of glyceryl monostearate and medium chain triglycerides (2.0% w/w to 10.0% w/w).

### Tonicity agents

Tonicity agents can be glycerol, mannitol, propylene glycol, ethylene glycol, sorbitol, etc. The concentration can be between 0.1% w/w and 5.0% w/w.

### Hydrophilic surfactants

Hydrophilic surfactants suitable for use in the present invention include anionic, non-anionic, cationic and amphoteric surfactants. Preferably, the surfactants of the present invention can be chosen from the group comprising, without limiting, non-ionic surfactants such as polysorbate 20, polysorbate 40, polysorbate 80, sorbitan monostearate 20, sorbitan monostearate 40, sorbitan monostearate 60, monostearate sorbitan 80, sodium cholate emulsifiers, sodium deoxycholate, sodium glycolate, poloxamers, sodium taurocholate, sodium taureodexicolate, and/or a mixture thereof.

In a preferred embodiment the formulations described in the present invention comprise the use of polysorbate 80 (1.0% w/w to 5.0% w/w).

### Polycations

The aqueous phase may contain non-lipid polycations such as chitosan, hexadimethrin bromide or other salt, poly-L-lysine, polyalylamine, polyethyleneimine, among others. The addition of these can be carried out during or after the formation of lipid nanostructures.

In a preferred embodiment the formulations described in the present invention comprise the use of chitosan (0.001 mg/mL w/v to 10 mg/mL w/v).

### Organic solvents

Organic solvents suitable for use in the present invention include, but are not limited to, protic and aprotic polar organic solvents, such as ethanol, acetone and/or a mixture thereof, and non-polar organic solvents, such as chloroform.

In a preferred embodiment the liposomes and nanoemulsions of the present invention comprise the use of chloroform for the solubilization of the components of the lipid phase and a mixture of chloroform: methanol: water (1:2.1:1).

### Obtaining lipid nanostructures

The processes for obtaining nanoemulsions include the steps of:
a) dissolving from 2.0% w/w to 20.0% w/w of the lipid phase in an organic solution composed of an organic solvent;
b) dissolving from 0.1% w/w to 5.0% w/w of tonicity agent in an aqueous solution;
c) evaporating all the organic solvent from the product obtained in step a, thus obtaining a lipid film;
d) adding the aqueous solution to the lipid film and leave for 4 to 72 hours at 2°C to 20°C;
e) sonicating the formulation for 1 to 60 minutes at a temperature between 25°C and 50°C;
f) homogenizing the formulation in a high-pressure homogenizer or microfluidizer, for 2 to 20 cycles of 250 to 2000 bar each;
g) adding non-lipid polycations (0.001 mg/mL w/v to 10 mg/mL w/v).

The processes for obtaining liposomes comprise the steps of:
a) dissolving from 2.0% w/w to 20.0% w/w of the lipid phase in an organic solution composed of an organic solvent;
b) dissolving from 0.1% w/w to 5.0% w/w of tonicity agent in an aqueous solution;
c) evaporating all the organic solvent from the product obtained in step a;
d) adding the aqueous solution to the lipid film and leave for 4 to 72 hours at 2°C to 20°C;
e) sonicating the formulation for 1 to 60 minutes at a temperature between 25°C and 50°C;
f) homogenizing the formulation in a high-pressure homogenizer or microfluidizer, for 2 to 20 cycles of 250 to 2000 bar each;
g) extruding the formulation on at least one membrane with a pore from 1000 nm to 220 nm and on at least one membrane from 220 nm to 50 nm.
h) adding non-lipid polycations (0.001 mg/mL w/v to 10 mg/mL w/v).

An additional object of the present invention is the processes for obtaining nanoemulsions that will have encapsulated nucleic acids, with the steps of:
a) dissolving from 0.1% w/w to 5.0% w/w of cationic lipid together with nucleic acids in a monophasic mixture of non-polar:protic:protic solvents (1:2.1:1) for 30 minutes;
b) separating the monophase in a two-phase system by adding a non-polar solvent and a protic one (2 mL each), followed by a brief vortex. The polar and non-polar phases are separated by centrifugation at a pressure between 1000 and 4000 x g for 2 to 30 min at a temperature between 15°C and 35°C;
c) adding 2.0% w/w to 20.0% w/w of lipid phase to the organic phase of the pre-complex;
d) dissolving from 0.1% w/w to 5.0% w/w of tonicity agent in an aqueous solution;
e) evaporating the organic solvent to form a film;
f) adding the aqueous phase;
g) leaving for 4 to 72 hours at 2°C to 20°C;
h) sonicating for 1 to 60 minutes at a temperature between 25°C and 50°C;
i) homogenizing the formulation in a high-pressure homogenizer or microfluidizer, for 2 to 20 cycles of 250 to 2000 bar each;
j) adding non-lipid polycations (0.001 mg/mL w/v to 10 mg/mL w/v).

The process of obtaining solid lipid nanostructures and nanostructured lipid carriers containing the adsorbed nucleic acids comprises the steps of:
a) melting from 2.0% w/w to 20.0% w/w of lipid phase at a temperature between 30°C to 80°C;
b) dissolving 1.0% w/w 5.0% w/w of surfactant and 0.1% w/w 5.0% w/w of tonicity agent in an aqueous solution, with a temperature of 30°C to 80°C;
c) adding the aqueous solution to the oily solution, under agitation and at a temperature of 30°C to 80°C;
d) stirring in the disperser ultra-turrax, at a speed between 500 and 25000 rpm under heating from 30°C to 80°C, for a period from 30 seconds to 5 minutes;
e) homogenizing the formulation in high-pressure homogenizer or microfluidizer, for 2 to 20 cycles of 250 to 2000 bar each.
f) adding non-lipid polycations (0.001 mg/mL w/v to 10 mg/mL w/v).

### Formulations comprising lipid carriers

The products of the present invention are formulations that comprise lipid nanostructures, associated with suitable excipients, useful in the pharmaceutical and medical fields.

The carriers of the present invention can be in the form of nanoemulsions, liposomes, solid lipid nanoparticles and nanostructured lipid carriers.

In one embodiment, the compositions may be incorporated in the form of a solution, suspension, gel, powder, among others.

### Specification of lipid nanoparticles

The formation of nanoparticles was first confirmed by evidence of a homogeneous character (without phase separation) and by the absence of precipitates. Next, the formulations were specified according to the average droplet/vesicle diameter, polydispersity index, zeta potential, and ability to complex with nucleic acids.

Droplet/particle diameter and polydispersity index (PDI) determination:

The formulations were specified through the spreading of dynamic light by the diffusion of monochromatic laser beam that crosses the colloidal dispersion. This determination was made by observing the scattering at 173°C after diluting the samples in purified water, previously filtered through a 0.22 µm membrane. The results were expressed as an average of three independent determinations.

### Determination of zeta potential

The zeta potential was determined through the electrophoretic mobility of the droplets/vesicles. The measurements were performed after calibration with a standard solution at -55 mV (polystyrene carboxylate latex). All analyzes were performed after diluting the samples in purified water, previously filtered through a 0.22 µm nylon membrane. The results were expressed as an average of three independent determinations.

### Complexation rate with nucleic acids

The complexation of nucleic acids with the formulations was verified by electrophoresis on agarose gel. The complexes were evaluated at a +4/-1 charge ratio (cationic lipid charges/nucleic acid charges) and were subjected to electrophoresis on a 1% agarose gel stained with the SYBR® Gold Nucleic Acid Gel Stain dye (Invitrogen, Carlsbad, USA). The stability of cationic nanostructures/DNA complexes was determined using a digestion assay with DNase I (Invitrogen, Carlsbad, USA). The bands were analyzed, and their intensity was calculated using the ImageJ® software, generating the percentage complexation rate.

### Example 1: Lipid nanocarrier consisting of a nanoemulsion with complexation of nucleic acid by adsorption.

### Final composition:

Lipid phase
   a. 5% w/w Medium chain triglycerides
   b. 0.56% w/w DOPE
   c. 0.56% w/w DOTAP
   d. 0.285% w/w DSPE-PEG
Aqueous phase
   e. 2.25% w/w Glycerol
   f. nucleic acids for the +4/-1 ratio (DOTAP/NUCLEIC ACID).
   g. 0.001 mg/mL w/v chitosan.

### Procedure:

First, the components of the lipid phase were weighed and dissolved in chloroform, with constant stirring. The components of the aqueous phase were weighed and dissolved in purified water, with constant stirring. The organic phase was route-evaporated at normal pressure and room temperature, to eliminate the organic solvent and to total dryness, to form the lipid film. At the end of the process, the aqueous phase was poured over a lipid film, which was maintained at 4°C for 12 hours. Afterwards, the formulation was sonicated at 37°C for 15 minutes. Then, the formulation was homogenized in a high-pressure homogenizer for 10 cycles of 500 bar each, in order to keep the droplet diameter of the oil phase as small as possible and with a lower polydispersity index. Finally, the nucleic acids were added to the formulation and then, the chitosan solution.

### Product obtained: Nanoemulsion (NA)

### Results:

- Size: 163 nm
- IPD: 0.14
- Zeta potential: +47,1 mV
- Complexation rate: 100%. See figure 1.

### Example 2: Lipid nanocarrier consisting of a nanoemulsion with nucleic acid complexation by encapsulation.

### Composition:

Lipid phase
   h) 5% w/w Medium chain triglycerides
   i) 0.56% w/w DOPE
   j) 0.56% w/w DOTAP
   k) 0.285% w/w DSPE-PEG
Aqueous phase
   l) 1. 2,25% w/w glycerol
   m) nucleic acids for the +4/-1 ratio (DOTAP/NUCLEIC ACID)
   n) 0.001 mg/mL w/v chitosan.

### Procedure:

First, the components of the lipid phase were weighed. The hydrophobic DNA/DOT AP complex was prepared by incubating the nucleic acids with the cationic lipid DOTAP in a monophasic mixture of chloroform:methanol:water (1:2.1:1) at room temperature for 30 min. The monophase was then divided into two phases by adding chloroform and water (2 mL each), followed by a brief vortex. The upper aqueous and lower organic phases were separated by centrifugation at 2000 x g for 10 min at room temperature. In the organic phase, then, the other lipids were dissolved. The components of the aqueous phase were weighed and dissolved in purified water, under constant stirring. The organic phase was route-evaporated at normal pressure at room temperature, to eliminate the organic solvent up to total dryness, to form the lipid film. At the end of the process, the aqueous phase was poured over the lipid film, which is maintained at 4°C for 12 hours. Afterwards, the formulation was sonicated at 37°C for 15 minutes. Then, the formulation was homogenized in a high-pressure homogenizer for 10 cycles of 500 bar each, in order to keep the droplet diameter of the oil phase as small as possible and with a lower polydispersity index. Finally, the chitosan solution was added to the formulation. Product obtained: Nanoemulsion (NE).

### Results

- Size: 163 nm
- IPD: 0.14
- Zeta potential: +48.7 mV
- Complexation rate: 100%. See figure 1.

### Example 3: Lipid nanocarrier consisting of a liposome with complexation of nucleic acid by adsorption.

### Composition:

Lipid phase
   o) 0.56% w/w DOPE
   p) 0.56% w/w DOT AP
   q) 0.285% w/w DSPE-PEG
Aqueous phase
   r) 2.25% w/w Glycerol
   s) nucleic acids for the + 4/-1 ratio (DOTAP/NUCLEIC ACID)
   t) 0.001 mg/mL w/v chitosan.

### Procedure:

First, the components of the lipid phase were weighed and dissolved in chloroform, with constant stirring. The components of the aqueous phase were weighed and dissolved in purified water, with constant stirring. The organic phase was route-evaporated at normal pressure and room temperature, to eliminate the organic solvent up to total dryness, to form the lipid film. At the end of the process, the aqueous phase is poured over the lipid film, which is maintained at 4°C for 12 hours. Afterwards, the formulation is sonicated at 37°C for 15 minutes. Then, the formulation is homogenized in a high-pressure homogenizer for 10 cycles of 500 bar each, in order to keep the droplet diameter of the oil phase as small as possible and with a lower polydispersity index. Finally, nucleic acids were added to the formulation and, afterwards, chitosan.

Product obtained: Liposome (LA).

### Results:

- Size: 108 nm
- IPD: 0.17
- Zeta potential: +38.7 mV
- Complexation rate: 100%. See figure 1.

### Example 4: Lipid nanocarrier consisting of a solid lipid nanoparticle with complexation of nucleic acid by adsorption.

### Composition:

Organic phase
   u. 1.4% w/w Glyceryl monostearate
   v. 0.6% w/w DOT AP
Aqueous phase
   w. 1.0% w/w Polysorbate 80
   x. 2.25% w/w Glycerol
   y. nucleic acids for the + 4/-1 ratio (DOTAP/NUCLEIC ACID)
   z. 0.005 mg/mL w/v chitosan.

### Procedure:

First, the components of the lipid phase were weighed and melted at a temperature of 80°C, under constant stirring. The components of the aqueous phase were weighed and dissolved in a final volume of 100 mL of purified water, under constant stirring at a temperature of 80°C. The aqueous phase was poured over the lipid phase, maintaining the temperature at 80°C and with constant stirring for 15 minutes. The formulation was mixed in ultra-turrax for 1 minute, at a speed of 13,500 rpm and a temperature of 80°C. At the end of the process, the formulation was then homogenized in a high-pressure homogenizer with 10 cycles of 750 bar each, in order to keep the particle diameter of the oil phase as small as possible and with a lower polydispersity index. The product was left to stand at room temperature for at least 10 minutes. Finally, the chitosan solution was added.

Product obtained: Solid lipid nanoparticle.

### Results:

- Size: 388 nm
- IPD: 0.69
- Zeta potential: +2.20 mV
- Complexation rate: 100%.

### Example 5: Lipid nanocarrier consisting of a nanostructured lipid carrier. Composition

Organic phase
   aa.1, 0% w/w Glyceryl monostearate
   bb.0,5% w/w DOTAP
   cc. 0.5% w/w medium chain triglycerides
Aqueous phase
   dd. 1, 0% w/w Polysorbate 80
   ee. 2,25% w/w Glycerol
   ff. nucleic acids for the + 4/-1 ratio (DOTAP/NUCLEIC ACID)
   gg. 0.05 mg/mL w/v chitosan.

### Procedure:

First, the components of the lipid phase were weighed and melted at a temperature of 80°C, under constant stirring. The components of the aqueous phase were weighed and dissolved to a final volume of 100 mL of purified water, with constant stirring at a temperature of 80°C. The aqueous phase was poured over the lipid phase, maintaining the temperature at 80°C and with constant stirring for 15 minutes. The formulation was mixed in ultra-turrax for 1 minute, at a speed of 13,500 rpm and a temperature of 80°C. At the end of the process, the formulation was then homogenized in a high-pressure homogenizer with 10 cycles of 750 bar each, in order to keep the particle diameters of the oil phase as small as possible and with a lower polydispersity index. The product was left to stand at room temperature for at least 10 minutes. Finally, chitosan was added.

Product obtained: Nanostructured lipid carrier.

### Results:

- Size: 238 nm
- IPD: 0.48
- Zeta potential: +3, 12 mV
- Complexation rate: 100%.

### Nasal administration

For the delivery of lipid carriers containing at least one nucleic acid for the purposes of gene therapy of the central nervous system, the invention provides nasal administration *in vivo.* This administration can be done with nasal drops, by intranasal or intratracheal spray for pulmonary and/or cerebral delivery, by inhalation, and/or through other aerosol vehicles.

For the delivery of lipid carriers containing at least one nucleic acid for purposes of gene therapy of the central nervous system, the invention prioritizes the use of the nasal route. Many potential drugs for the treatment of neurological diseases are unable to reach the brain in sufficient concentrations to be therapeutic due to the blood-brain barrier. On the other hand, the direct administration of drugs to the brain provides the possibility of greater therapeutic effectiveness than with the systemic administration of a drug, precisely by working around the blood-brain barrier and providing the transport of high molecular weight molecules. The use of nasal administration of therapeutic agents to the brain provides a means of working around the blood-brain barrier in a non-invasive way. In this context, nanometric drug carriers have been shown to improve the delivery of drugs to the central nervous system compared to equivalent drug solutions. Neurological conditions that could benefit from nasal administration for delivery to the brain include pain, epilepsy, neurodegenerative diseases and infectious diseases (WY, O. et al, Nose-to-brain drug delivery by nanoparticles in the treatment of neurological disorders, Curr Med Chem, 2014, 21 (37), p. 4247-56).

### Application examples of the product obtained according to example 3, described above:

### In vivo assays in a murine MPS I model

A knockout mouse animal model for the Idua (murine) gene was used. This model was created by interrupting exon 6 of the Idua gene. In the middle of the exon, a neomycin resistance gene was inserted in reverse direction. As a result, mice were produced with a disease that mimics Hurler's Syndrome, the most severe phenotype of MPS I, with increased levels of glycosaminoglycans in the urine and in various tissues, and undetectable activity of Idua.

All procedures with animals were carried out at the Animal Experimentation Unit of the Experimental Research Center of Hospital de Clínicas de Porto Alegre, and follow the norms of adequacy to the current guidelines set forth in Law 11.794/08 and in normative resolutions number 12 (Use of Animals for Scientific and Didactic purposes) and 30 (CONCEA's Euthanasia Practice Guidelines). The animals are kept in a controlled environment (temperature 20-24°C, air relative humidity 40-60% and air exhaust systems) with cycles of 12 hours of light and 12 hours of darkness and standard commercial food for the species and water ad libitum. All animals used were genotyped by molecular identification of the transgene.

### Construction of plasmids CRISPR/Cas9 ROSA26 and donor Idua ROSA26

A plasmid from the CRISPR/Cas9 system was used for genomic editing experiments. In this system, the Cas9 nuclease and the guide RNA formed by a crRNA-tracrRNA transcript are present in a single vector, sgRNA (single guide RNA). A target sequence for cleavage by Cas9 was selected at the ROSA26 locus of the mouse genome and was inserted into the vector. The complete vector was inserted by thermal shock transformation into TOP 10 competent bacteria (Invitrogen, USA), whose colonies were then expanded and subjected to plasmid extraction with the Maxiprep kit (Life Technologies, USA). The extracted plasmideal DNA was then sequenced to verify the correct orientation of the insert.

For targeted recombination, a vector containing the cDNA of Idua is used. The construct contains the cDNA sequence of Idua regulated by a promoter and two homologous regions (approximately 1 kb each) to the mouse ROSA26 locus, in the locus region where Cas9 recognizes and cleaves.

For the gene therapy experiments of the central nervous system with recombinant plasmid, a plasmid (pIDUA) containing the cDNA of IDUA was constructed using the commercial expression vector pREP9 (Invitrogen, USA) as described by Camassola et al. (M. Camassola , L.M. Braga, A. Delgado-Cafíedo, T.P. Dalberto, U. Matte, M. Burin, R. Giugliani, N.B. Nardi, Nonviral in vivo gene transfer in the mucopolysaccharidosis I murine model, J. Inherit. Metab. Dis. 28 (2005) 1035-1043).

### Treatments

One group (n = 5) was used for the administration of the liposomal complexes containing the plasmid CRISPR/Cas9 and the plasmid Idua/Rosa26 (LA) and this received the LA complex nasally in 30 applications of 120µL. As controls, normal mice that received no treatment (n = 3) and MPS I mice that received no treatment (n = 3) were used. For nasal administration, the animals are immobilized by the researcher and six doses of 10 µL are instilled in each nostril, every 15 minutes, once a day, for 30 days.

Another treatment was carried out using a group (n = 5) for the administration of the nanoemulsion complexes containing the plasmid pIDUA (NA/pIDUA) and this received the complex nasally in 2 applications of 120µL. As controls, normal mice that received no treatment (n = 3) and MPS I mice that received no treatment (n = 3) were used. For nasal administration, the animals are immobilized by the researcher and six doses of 10 µL are instilled in each nostril, every 15 minutes, twice in a day.

### Serum enzymatic dosage of Idua

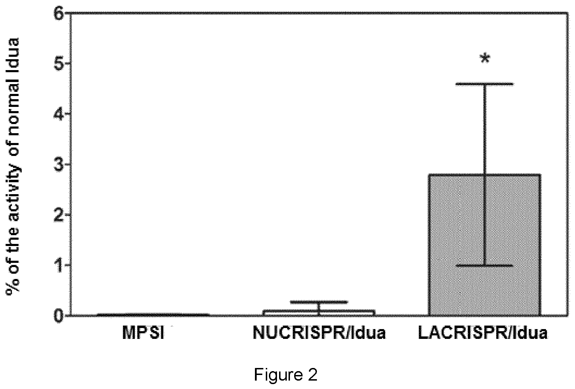

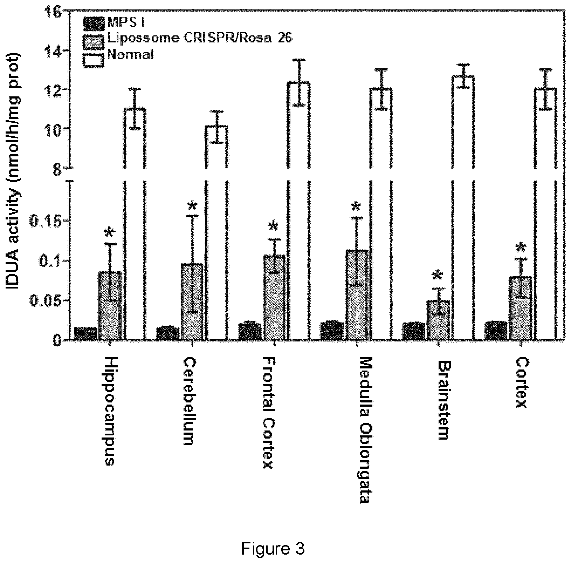

The serum and tissue level of Idua was measured in animals treated with LA from 15 days after treatment and after 30 days. The results were compared with untreated MPS I animals and normal animals. The enzymatic activity was evaluated through the enzymatic assay by fluorimetric method using the artificial substrate 4-methyl-umbeliferyl-alpha-Liduronide. The unit to be adopted was nmol/h/mL of serum or nmol/h/mg of protein (measured using the Lowry method). For this, the serum was incubated with the fluorescent substrate 4-methylumbelliferyl a-L-iduronide at 37°C for 1 h in sodium formate buffer (pH 2.8). Fluorescence was measured at 365 nm (excitation) and 450 nm (emission) using the SpectraMax M2 fluorimeter (Molecular Devices, CA, USA). In this test, the amount of IDUA was measured by the amount of substrate cleaved in 1 hour. See Figures 2 and 3.

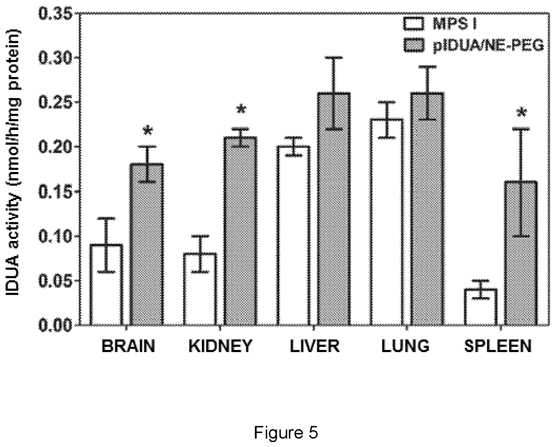

Figure 5 shows the IDUA enzyme activity values found in different organs and more precisely in the brain of untreated MPS I mice and in MPS I mice treated with the NA/pIDUA complex nasally in one application. Values related to the enzymatic activity of normal mice.

### Equivalents

Various modifications of the invention and many other embodiments thereof, in addition to those depicted and described herein, will become apparent to those skilled in the art of the entire content of this document, including references to the scientific and patent literature referred to herein. The present subject contains important information, exemplification and guidance that can be adapted to the practice of this invention in its various embodiments and equivalents.

## Claims

1. Composition for gene therapy of the central nervous system **characterized in that** it comprises at least one adsorbed or encapsulated nucleic acid and non-viral carriers, with average droplet/particle diameter in the range of 0.001 to 1.0 micrometer.

2. Composition for gene therapy of the central nervous system, according to claim 1, **characterized in that** the nucleic acids are one or more selected from the group consisting of: guide RNA sequence, nuclease coding sequence, model DNA sequence for homologous recombination, sequence of a recombinant gene or plasmid containing the entire sequence of a gene.

3. Composition for gene therapy of the central nervous system, according to any of claims 1 and 2, **characterized in that** the nanostructures are nanoemulsions, liposomes, solid lipid nanoparticles or nanostructured lipid carriers.

4. Composition for gene therapy of the central nervous system according to any one of claims 1 to 3, **characterized in that** it comprises pharmaceutically suitable excipients.

5. Process for obtaining a composition for gene therapy of the central nervous system as defined in any one of claims 1 to 4, **characterized in that** obtaining the composition for gene therapy of the central nervous system comprises the steps of:
(a) dissolving between 2.0% w/w to 20.0% w/w of lipid phase in an organic solution;
(b) dissolving between 0.1% w/w to 5.0% w/w of tonicity agent in an aqueous solution;
(c)evaporating the organic solution obtained in step (a), to form a film;
(d) adding the aqueous solution obtained in step (b) to the lipid film obtained in step (c);
(e) letting the product obtained in step (d) rest for 4 to 72 hours at a temperature between 2°C and 20°C;
(f) sonicating the formulation obtained in step (e) for 1 to 60 minutes at a temperature between 25°C and 50°C;
(g) homogenizing the formulation obtained in step (f) in a high-pressure homogenizer or microfluidizer, for 2 to 20 cycles of 250 to 2000 bar each; and
(h) adding non-lipid polycations (0.001 mg/mL w/v to 10 mg/mL w/v).

6. Process for obtaining a composition for gene therapy of the central nervous system according to claim 7, if the product to be obtained is the liposome, **characterized in that** it comprises the additional step:
(i) extruding the formulation obtained in step (g) at least one membrane with pore size from 1000 nm to 220 nm and at least one membrane with pore size from 220 nm to 50 nm.

7. Process for obtaining a composition for gene therapy of the central nervous system, according to claim 6, **characterized in that** the organic solution is an organic solvent chosen from the group comprising of polar protic, aprotic or non-polar organic solvents and/or mixture thereof.

8. Process for obtaining a composition for gene therapy of the central nervous system according to claim 6, **characterized in that**, to obtain nanoemulsions containing the encapsulated nucleic acids, the organic solution described in step (a) is the organic phase content of the pre-complex obtained through the steps:
i. dissolving from 0.1% w/w to 5.0% w/w of cationic lipid and nucleic acids in a monophasic mixture of nonpolar:protic:protic solvents (1:2.1:1) for 30 minutes;
ii. adding to the product obtained in step (i) 2 mL of non-polar solvent and 2 mL of protic solvent;
iii. stirring the product obtained in step (ii) briefly in a vortex;
iv. centrifuging the product obtained in step (iii) at a pressure between 1000 and 4000 x g for 2 to 30 min at a temperature between 15 to 35°C; and
v. separating the organic phase obtained in step (iv).

9. Process for obtaining a composition for gene therapy of the central nervous system, as defined in any one of claims 1 to 4, **characterized in that**, in case the product to be obtained is solid lipid nanostructures or nanostructured lipid carriers containing the adsorbed nucleic acids, steps of:
(A) melting from 2.0% w/w to 20.0% w/w of lipid phase at a temperature between 30°C and 80°C;
(B) dissolving 1.0% w/w 5% w/w surfactant and 0.1% w/w 5.0% w/w of a tonicity agent in an aqueous solution, with a temperature of 30°C to 80°C;
(C) adding the aqueous solution from step (A) to the oily solution from step (B), under stirring and at a temperature of 30° to 80°C;
(D) stirring the product obtained in (C) in disperser ultra-turrax, at a speed between 500 and 25000 rpm, under heating from 30°C to 80°C, for 30 seconds to 5 minutes;
(E) homogenizing the formulation obtained in (D) in homogenizer at high-pressure or microfluidizer, for 2 to 20 cycles of 250 to 2000 bar each; and
(F) adding non-lipid polycations (0.001 mg/mL w/v to 10 mg/mL w/v).

10. Process for obtaining a genome editor composition, according to claims 5 to 9, **characterized in that** the formulation is subjected to a later stage of evaporation of water under normal or reduced pressure between 0 and 1000 mbar at a temperature between 10°C and 50°C.

11. Process for obtaining a composition for gene therapy of the central nervous system, according to claim 9, **characterized in that** the protic polar organic solvent is methanol, and the non-polar organic solvent being chloroform.

12. Process for obtaining a composition for gene therapy of the central nervous system, according to claims 5 to 9, **characterized in that** the lipid phase is chosen from the group consisting of:
a) liquid lipids such as decyl oleate, isohexadecane, stearic and/or oleic acid esters, coconut fatty acid ethanolamide, natural oils such as corn, peanuts, sesame, olive, jojoba, soy, fatty alcohol, paraffin, medium chain triglycerides, long chain triglycerides, palmitates, myristates and octyldodecanol;
b) solid lipids such as tristearin, tricaprine, trilaurine, trimiristin, tripalmitin, stearic acid, cetyl alcohol, stearyl alcohol, cocoa butter, carnauba wax, beeswax, cetyl palmitate, glyceryl monostearate, glycerol behenate, palmitate glyceryl, glyceryl tripalmitate, glyceryl trimiristate, glyceryl tristearate and/or a mixture thereof;
c) lipophilic surfactants such as lecithins and phospholipids and/or a mixture thereof;
d) neutral lipids;
e) cationic lipids; and
f) PEG branching lipids (pegylated)

13. Process for obtaining a composition for gene therapy of the central nervous system, according to claims 5 to 9, **characterized in that** the tonicity agent is chosen from the group comprising sorbitol, ethylene glycol, polyethylene glycol, mannitol, glycerol, and/or mixture thereof.

14. Process for obtaining a composition for gene therapy of the central nervous system, according to any one of claims 5 to 13, **characterized in that** the lipid phase and the aqueous phase of the process of obtaining the liposomes comprise:
Lipid phase:
- DOPE (0.5% w/w to 5.0% w/w);
- DOTAP (0.5% w/w to 5.0% w/w);
- DSPE-PEG (0.25% w/w to 5.0% w/w);
Aqueous phase:
- Glycerol (0.1% w/w and 5.0% w/w);
- Nucleic acids for the proportion between + 2/-1 and + 8/-1 (DOTAP/NUCLEIC ACID);
- Solution of non-lipid polycations (0.001 mg/mL w/v to 10 mg/mL w/v).

15. Process for obtaining a composition for gene therapy of the central nervous system, according to any one of claims 5 to 13, **characterized in that** the lipid phase and the aqueous phase of the process for obtaining the nanoemulsions comprise:
Lipid phase:
- DOPE (0.5% w/w to 5.0% w/w);
- DOTAP (0.5% w/w to 5.0% w/w);
- DSPE-PEG (0.25% w/w to 5.0% w/w);
- Medium chain triglycerides (2.0% w/w to 20.0% w/w);
Aqueous phase:
- Glycerol (O, 1% w/w and 5.0% w/w)
- Nucleic acids for the proportion between + 2/-1 and + 8/-1 (DOTAP/NUCLEIC ACID);
- Solution of non-lipid polycations (0.001 mg/mL w/v to 10 mg/mL w/v).

16. Process for obtaining a composition for gene therapy of the central nervous system, according to any one of claims 5 to 13, **characterized in that** the lipid phase and the aqueous phase of the process for obtaining solid lipid nanoparticles comprise:
Lipid phase:
- glyceryl monostearate (2.0% w/w to 10.0% w/w);
- DOTAP (0.5% w/w to 5.0% w/w);
Aqueous phase:
- 1.0% w/w Polysorbate 80 (1.0% w/w to 5.0% w/w);
- Glycerol (O, 1% w/w and 5.0% w/w); and
- Nucleic acids for the proportion between + 2/-1 and + 8/-1 (DOTAP/NUCLEIC ACID);
- Solution of non-lipid polycations (0.001 mg/mL w/v to 10 mg/mL w/v).

17. Process for obtaining a genome editor composition, according to any one of claims 5 to 13, **characterized in that** the lipid phase and the aqueous phase of the process for obtaining nanostructured lipid carriers comprising:
Lipid phase:
- Mixture in the 7: 3 ratio of glycerol monostearate and medium chain triglycerides (2.0% w/w to 10.0% w/w);
- DOTAP (0.5% w/w to 5.0% w/w);
Aqueous phase:
- 1.0% w/w Polysorbate 80 (1.0% w/w to 5.0% w/w);
- Glycerol (0.1% w/w and 5.0% w/w); and
- Nucleic acids for the proportion between + 2/-1 and + 8/-1 (DOTAP/NUCLEIC ACID);
- Solution of non-lipid polycations (0.001 mg/mL w/v to 10 mg/mL w/v).

18. Process for obtaining a composition for gene therapy of the central nervous system, according to any one of claims 5 to 13, **characterized by** the addition of a solution of non-lipid polycations in the concentration of 0.001 mg/mL (w/v) to 10 mg/mL (w/v), which comprises chitosan, hexadimethrin bromide or other salt, poly-L-lysine, polyalylamine, polyethyleneimine, among others, and/or a mixture of these. The addition of these can be carried out before or after the formation of lipid nanostructures or complexes with nucleic acids.

19. Process for obtaining a composition for gene therapy of the central nervous system, according to any one of claims 5 to 18, **characterized by** the incorporation of the compositions in the form of solution, suspension, gel, powder, among other pharmaceutical forms.

20. Use of the composition for gene therapy of the central nervous system as defined in any of claims 1 to 4, **characterized in that** it is in the preparation of a medicine for the treatment of diseases caused by deficiencies or genetic abnormalities that have neurological impairment.

21. Use of the composition for gene therapy of the central nervous system according to claim 20, **characterized in that** it is in the preparation of a medication for the treatment of deposit lysosomal diseases.

22. Use of composition for gene therapy of the central nervous system according to claim 20 or 21, **characterized in that** the administration is nasal targeting the central nervous system.
